(19) Europäisches Patentamt
European Patent Office
Office européen des brevets

(11) Veröffentlichungsnummer: 0 178 600
B1

# (12) EUROPÄISCHE PATENTSCHRIFT

(45) Veröffentlichungstag der Patentschrift: 29.11.89

(51) Int. Cl.⁴: A 61 K 37/02 // (A61K37/02, 31:505)

(21) Anmeldenummer: 85112894.2

(22) Anmeldetag: 11.10.85

**E R R A T U M**

| DIE TEXTSTELLE :<br>TEXT PUBLISHED :<br>LE PASSAGE SUIVANT : | (SEITE, SPALTE, ZEILE)<br>(PAGE, COLUMN, LINE)<br>(PAGE, COLONNE, LIGNE) | | | LAUTET BERICHTIGT:<br>SHOULD READ :<br>DEVRAIT ETRE LU : |
|---|---|---|---|---|
| 7. Präparat nach einem der Ansprüche 5 oder 6, dadurch gekennzeichnet, dass es $10^{-6}$ bis $10^{-5}$ Mol eines Tetrahydrobiopterins und 1 - 20 Units/ml eines Lymphokins enthält. | 5 | 7 | 49/52 | 7. Verfahren nach einem der Ansprüche 5 oder 6, dadurch gekennzeichnet, dass es $10^{-6}$ bis $10^{-5}$ Mol eines Tetrahydrobiopterins und 1 - 20 Units/ml eines Lymphokins verwendet. |

Tag der Entscheidung über die Berichtigung
Date of decision on rectification:
Date de décision portant sur modification: 15.01.90

Ausgabe- und Veröffentlichungstag:
Issue and publication date:
Date d'edition et de publication: 14.03.90

Patbl.Nr:
EPB no: 90/11
Bull. no:

(19) Europäisches Patentamt
European Patent Office
Office européen des brevets

(11) Veröffentlichungsnummer: **0 178 600**
**B1**

# (12) EUROPÄISCHE PATENTSCHRIFT

(45) Veröffentlichungstag der Patentschrift: **29.11.89**

(51) Int. Cl.⁴: **A 61 K 37/02** // (A61K37/02, 31:505)

(21) Anmeldenummer: **85112894.2**

(22) Anmeldetag: **11.10.85**

(54) **Verwendung einer Kombination aus Tetrahydrobiopterinen und Lymphokinen zur Herstellung eines Arzneimittels mit gesteigerter in vivo und in vitro Lymphokin-Aktivität.**

(30) Priorität: **17.10.84 DE 3437944**

(43) Veröffentlichungstag der Anmeldung: **23.04.86 Patentblatt 86/17**

(45) Bekanntmachung des Hinweises auf die Patenterteilung: **29.11.89 Patentblatt 89/48**

(84) Benannte Vertragsstaaten: **AT BE CH DE FR GB IT LI LU NL SE**

(56) Entgegenhaltungen:
**CHEMICAL ABSTRACTS, Band 100, Nr. 15, 9. April 1984, Seite 432, Nr. 119130r, Columbus, Ohio, US; I. ZIEGLER et al.: "Pterins as functioning constituents of the lymphokine system", & BIOCHEM. CLIN. ASPECTS PTERIDINES 1983, 2(PROC. WINTER WORKSHOP PTERIDINES, 2ND) 185-93**
**CHEMICAL ABSTRACTS, Band 100, Nr. 15, 9. April 1984, Seite 433, Nr. 119137y, Columbus, Ohio, US; I. ZIEGLER et al.: "Participation of pterins in the control of lymphocyte stimulation and lymphoblast proliferation", & CANCER RES. 1983, 43(11), 5356-9**
**CHEMICAL ABSTRACTS, Band 103, Nr. 5, 5. August 1985, Seite 398, Nr. 36041y, Columbus, Ohio, US; I. ZIEGLER et al.: "Modulation of interleukin 2 activity by lymphocyte-derived tetrahydrobiopterin", & NATURWISSENSCHAFTEN 1985, 72(6), 330-1**

(73) Patentinhaber: **Biotest Aktiengesellschaft, Flughafenstrasse 4, D-6000 Frankfurt 71 (DE)**
Patentinhaber: **GESELLSCHAFT FÜR STRAHLEN- UND UMWELTFORSCHUNG M.B.H., Ingolstädter Landstrasse 1 Post Oberschleissheim, D-8042 Neuherberg (DE)**

(72) Erfinder: **Ziegler, Irmgard, Richildenstrasse 74, D-8000 München 19 (DE)**
Erfinder: **Schwulera, Udo, Dr., Krebsbachweg 23, D-6450 Hanau (DE)**
Erfinder: **Sonneborn, Hans H., Dr., Im Birkeneck 70, D-6056 Heusenstamm (DE)**

(74) Vertreter: **Wolff, Hans Joachim, Dr.jur. Dipl.-Chem. et al, Beil, Wolff & Beil Rechtsanwälte Postfach 80 01 40 Adelonstrasse 58, D-6230 Frankfurt am Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft den in den Ansprüchen gekennzeichneten Gegenstand.

Lymphokine sind bestimmte Substanzen, die auf einen Antigen-Reiz hin von Lymphozyten sezerniert werden. Die auch als Mediatoren zellvermittelter Immunität bezeichneten Lymphokine spielen u.a. auch bei Entzündungen eine wichtige Rolle. Sie sind keine Antikörper, sondern biologisch hochaktive Faktoren (Hormone), die rezeptorspezifisch auf bevorzugte Zielzellen wirken. Zu ihnen gehören beispielsweise Interleukin-2, Interleukin-3, CSF (Kolonie-stimulierender Faktor) und B-Zell-Faktoren. Die Begriffe in der Immunologie sind je nach dem Stand der Wissenschaft Änderungen unterworfen. So kann sich auch der Begriff «Lymphokine» ändern. Der Begriff Lymphokine soll im Vorliegenden deshalb auch solche Blutfaktoren umfassen, die die vorstehend definierten Eigenschaften der Lymphokine besitzen, jedoch zum gegenwärtigen Zeitpunkt nicht als solche klassifiziert sind.

Interleukin-2, abgekürzt IL-2, das früher auch als T-Zellen-Wachstumfaktor (TCGF) bezeichnet wurde (Aarden, L.A. et al: Revised nomenclature for antigennonspecific T-cell proliferation and helper factors, J. Immunology 123 (1979) 1928–1929 (1); Morgan, D.A., F.W. Ruscetti und R.C. Gallo: Selective in vitro growth of T lymphoctes from normal human bone marrow, Science 193 (1976) 1007 (2)), ist ein Glycoprotein mit dem Molekulargewicht von etwa 15000 und einem isoelektrischen Punkt im Neutralbereich (Gillis, S., D.Y. Mochizuki, P.J. Coulon, S.H. Hefeneider, C.A. Rambthun, A.E. Gillis, M.B. Frank, C.S. Henney und J.D. Watson: Molecular characterization of Interleukin-2, Immunolog. Rev. 63 (1982) 167–209 (3)). Seine gelegentlich beschriebene molekulare Heterogenität wird auf Unterschiede in der Glykosilierung zurückgeführt (Robb, R.J. und K.A. Smith: Heterogeniety of human T-cell growth factor due to variable glycosilation, Molecular Immunol. 18 (1981) 1087–1094 (4)).

IL-2 gilt als das «zweite Signal» in der Immunantwort (Ruscetti, F.W. und R.C. Gallo: Human T Lymphocyte Growth Factor: Regulation of Growth and Function of T Lymphocytes, Blood 57 (1981) 379–394 (5) und Wagner, H., C. Hardt, K. Heeg, K. Pfitzenmaier, W. Solbach, R. Bartlet, H. Stockinger und M. Röllinghoff: In vivo and in vitro effects of Interleukin-2, Immunol. Rev. 51 (1980) 215–236 (6)) und stellt einen Regulationsfaktor dar, mit dessen Hilfe es möglich ist, normale aktivierte und neoplastische T-Zellen kontinuierlich in vitro zu züchten. Zu den Effektorzellen, die durch Zugabe von IL-2-Präparaten kloniert und in Langzeitkultur gehalten werden können, zählen: T-Helfer und -Suppressorzellen, zytotoxische T-Zellen und natürliche Killerzellen (NK-Zellen). Zahlreiche Störungen in der Immunabwehr (immunologische Krankheiten) sind auf das Fehlen von Interleukin-2-produzierenden Zellen bzw. einer zu geringen IL-2-Produktion der auf das nicht oder zu wenig Ausbilden von IL-2-Rezeptoren zurückzuführen und können in vielen Fällen durch Verabreichung von Interleukin-2 verbessert werden.

Neuere Untersuchungen haben ferner gezeigt, dass die Interleukin-2-Bildung mit zunehmendem Alter stark abnimmt. Mit Interleukin-2 ist somit ein therapeutisches Mittel an die Hand gegeben, durch das Krankheiten, die durch gestörte Interleukin-2-Produktion verursacht werden, geheilt oder gelindert werden.

Auch in der Krebstherapie kann IL-2 seine Anwendung finden.

Zur Diagnostizierung derartiger Zustände werden beispielsweise monoklonale Antikörper gegen Interleukin-2 hergestellt, um damit ein Diagnose-Kit auf Radioimmun-Assay oder Enzym-Immuno-Sorbent-Assay-Basis aufzubauen.

Pterine werden in der Literatur beschrieben als tierische Pigmente, Cofaktoren (Tetrahydrobiopterin) bei der Hydroxylierung von Tryptophan, Phenylalanin, Tyrosin (Fehlen führt zu schweren neurologischen Störungen, Hyperphenylalanimämie), Regulatoren der Zellproliferation (teilweise Ersatz von fötalem Kälber-Serum) und als Faktoren, die Einfluss auf das Immunsystem ausüben (Römpps Chemie-Lexikon, 7. Aufl., S. 2831–2832 (7); S. Milstein und S. Kaufman «Tetrahydro-Sepiapterin is an intermediate in tetrahydrobiopterin Biosynthesis», Biochemical and Biophysical research communications; Vol. 115, No. 3, September 30, 1983, S. 888–893 (8); Aus Chemical Abstracts 100: 119 137y, Ziegler et al und Chemical Abstracts 100: 119 130 r, Ziegler et al ist bekannt, dass Pterine eine regulierende Wirkung, teils positiv, teils negativ, auf T-Lymphozyten im Stadium der Aktivierung ausüben.

Der biologische Nachweis von Lymphokinen ist häufig, wenn sie in sehr kleinen Mengen vorliegen, sehr schwierig, wodurch die Diagnose erschwert wird.

Ausserdem hält man es für erstrebenswert, die Wirksamkeit von Lymphokinen bei der therapeutischen Anwendung zu steigern.

Der Erfindung liegt die Aufgabe zugrunde, die Nachweisgrenze im biologischen Lymphokin-Test sowie die in vitro und in vivo Aktivität von Lymphokin zu steigern.

Diese Aufgabe wird erfindungsgemäss dadurch gelöst, dass man Tetrahydrobiopterine mit Lymphokinen verwendet bzw. Lymphokinpräparate mit einem Zusatz eines Tetrahydrobiopterins versieht.

Zwar ist aus den beiden letztgenannten Druckschriften die teilweise positive regulierende Wirkung von Pterinen im Stadium der Aktivierung der T-Lymphozyten bekannt, jedoch war es überraschend, dass durch Zusatz eines Tetrahydrobiopterins zu einem Lymphokin die Aktivität von Lymphokinen um das 3- bis 5fache gesteigert werden kann und somit Tetrahydrobiopterine als Immunmodulatoren von Lymphokinen wirken, obgleich Tetrahydrobiopterine allein in diesem Stadium der T-Zell-Immunantwort keine Effekte zeigen.

Im Falle von Interleukin-2-ermöglicht der erfindungsgemässe Zusatz eines Tetrahydrobiopterins zu Interleukin-2 Laborpräparaten eine Steigerung der Nachweisgrenze und damit eine erhöhte Sensitivität des Testsystems. Ausserdem hat ein Zusatz von Tetrahydrobiopterinen einen positiven Einfluss auf die Vermehrung von aktivierten T-Zellen. Auch im diagnostischen Interleukin-2-Test wird bei der erfindungsgemässen Verwendung eines Tetrahydrobiopterins als Zusatz zum ELISA bzw. RIA Test-Kit für Interleukin-2 die Nachweisgrenze gesteigert.

Ferner hat der Zusatz eines Tetrahydrobiopterins zu therapeutischen Interleukin-2-Präparaten eine Aktivitätssteigerung, d.h. eine Steigerung der Immunabwehr, zur Folge. Dazu gehören:

Vermehrung von aktivierten T-Zellen, Steigerung der natürlichen Killer-Zell-Aktivität, Induktion von IL-2 abhängigen Killerzellen, Induktion von gamma-Interferon und Einfluss auf die Expression von D/Dr-Antigenen.

Wahrscheinlich wird auch ein positiver Einfluss auf B-Zellen ausgeübt.

Ohne sich auf eine bestimmte Theorie festzulegen, weisen die Pterin-Analysen in aktivierten Lymphozyten und andere Studien darauf hin, dass die Anwesenheit eines Glycoproteins Voraussetzung für die Pterinwirkung ist, was beim konventionell hergestellten Lymphozyten Interleukin-2 der Fall ist.

Als besonders bevorzugtes Tetrahydrobiopterin wird erfindungsgemäss L-erythro-Tetrahydrobiopterin verwendet, dessen Biosynthese wie folgt vonstatten geht:

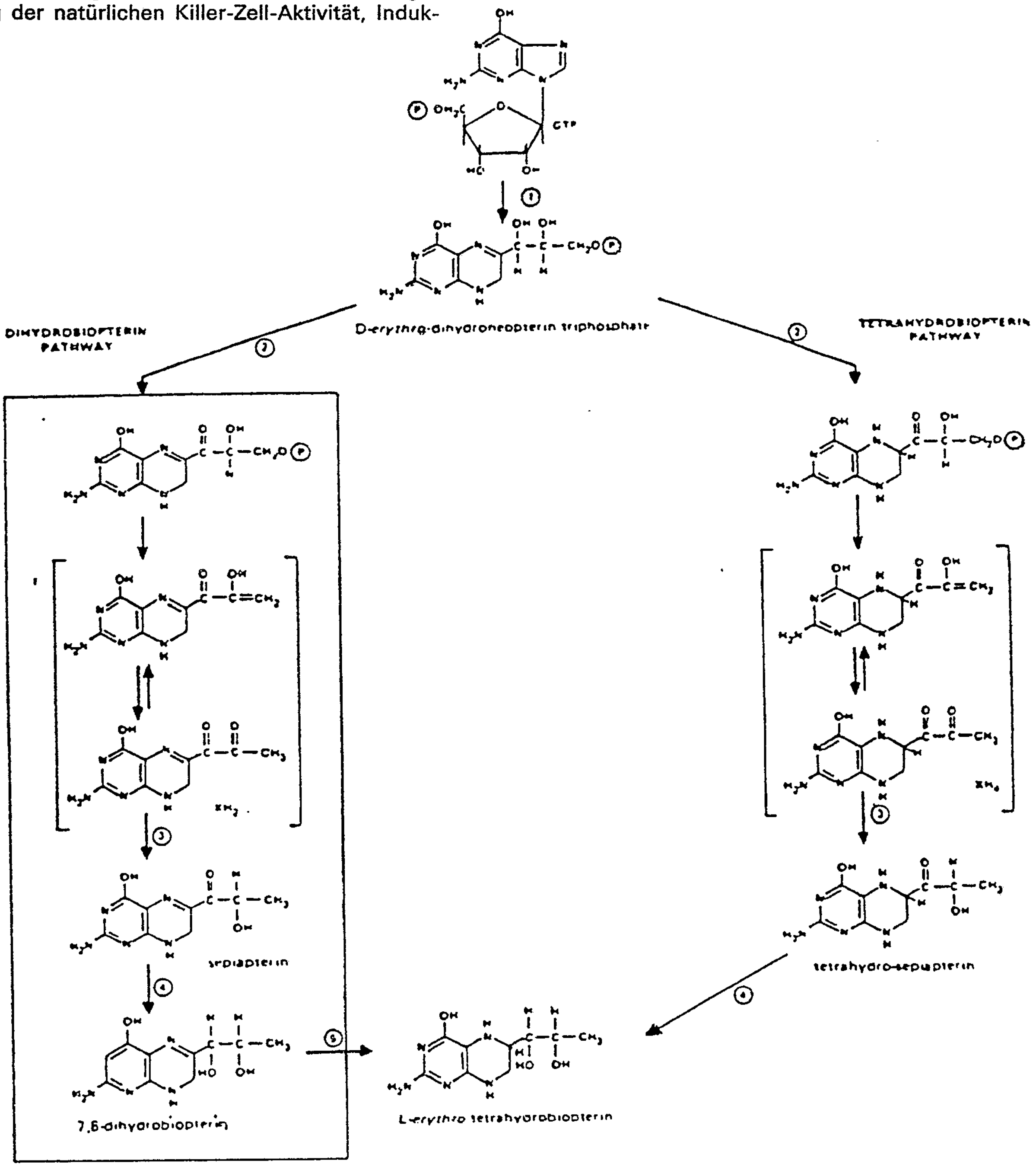


Biochemical and Biophysical Research Communications, Vol. 115, No. 3 (1983) 882, 892.

Bei den für die Tests auf aktivitätssteigernde Wirkung eines Tetrahydrobiopterins herangezogenen Lymphokinpräparaten handelt es sich um rohes Interleukin-2 (vgl. DE-OS 3149360, Beispiel 1); partiell gereinigtes Interleukin-2 (vgl. a.a.O. Beispiel 4); hochreines Interleukin-2 (vgl. Robb R.J. et al, Biochem. Biophys. Research COMM 116 (1983) 1049–1055, jedoch unter Verwendung eines anderen Antikörpers, nämlich des in der deutschen Patentanmeldung DE-A-3329449 beschriebenen Antikörpers gegen Interleukin-2) und inaktiviertes Interleukin-2, eine beim Lyophylisieren inaktiv gewordene Charge eines der nach vorstehenden Verfahren hergestellten aktiven Interleukin-2.

Überraschenderweise lässt sich erfindungsgemäss nicht nur eine Aktivitätssteigerung der aktiven Präparate erzielen, sondern es lassen sich auch bei der Präparation inaktiviert gewordene Produkte reaktivieren.

Die Steigerung der Aktivität von Lymphokinen, insbesondere von Interleukin-2, konnte bei Interleukin-2-abhängigen cytotoxischen T-Zell-Indikatorlinien der Maus sowie bei Maus-Milz-Lymphozyten bzw. Humanlymphozyten (mononukleäre Zellen), die mit ConA bzw. PHA stimuliert worden waren, als Indikatorzellen festgestellt werden.

Als Anzeichen der Steigerung der Aktivität gilt der Grad des $^3$H-Thymidineinbaus.

Aus der Gesamtheit der durchgeführten Versuche resultiert, dass ein Wirkungsoptimum bei einer Tetrahydrobiopterinkonzentration zwischen etwa $10^{-6}$ und $10^{-5}$ Mol erzielt wird bei einer Interleukin-2-Konzentration von etwa 1 bis 20 Units/ml.

Die Anzahl der Units der Interleukin-2-Aktivität berechnet sich aus einem Vergleich mit dem vorläufigen internationalen Standard. Die vorläufige internationale Reference Unit für Interleukin-2 ist in Lymphokine Research, 3. Aufl., 1984, beschrieben.

Zum Nachweis des Einflusses von Tetrahydrobiopterinen auf die Wirkung von Lymphokinen wurden nachstehende Versuche durchgeführt:

Beispiel 1

A. Herstellung von PHA-Blasten

Zu 100 ml Vollblut wurden 2000 IE Na-Heparin zugegeben, um die Gerinnung zu verhindern. Durch einen Gradienten über Ficoll-Hypaque (1:1) wurden die mononukleären Zellen (MNC) isoliert (400 x g, 20 Min.). Nach dem Waschen der Zellen wurden diese mit 0,5 µg PHA/ml 72 h stimuliert.

Ansatz: 0,8 × $10^6$ MNC/ml
0,5 µg PHA/ml
10% FCS
100 U Penicillin/ml
100 µg Streptomycin/ml
37 °C
6% $Co_2$
in RPMI 1640
(vgl. Cancer Research, 43, 5356–5359 (1983)

Danach wurden die Blasten abzentrifugiert, gewaschen und wie oben geschildert erneut 6 h inkubiert. Dann wurde erneut abzentrifugiert.

B. Die gemäss Stufe A gewonnenen Zellen wurden in das gleiche Medium wie in Stufe A aufgenommen, dem zusätzlich 10% FCS zugesetzt worden waren. 100 µm eines gemäss DE-OS 3149360, Beispiel 4, hergestellten Interleukin-2-Präparates wurden mit 100 µl einer wässrigen Lösung Tetrahydrobiopterin (1 µl einer wässrigen Lösung Tetrahydrobiopterin (1 µg Tetrahydrobiopterin/10 µl Medium bzw. 10 µl IL-2) versetzt und serial im Medium jeweils um die Hälfte verdünnt. 100 µl Zellen wurden mit 100 µl der serialen Verdünnung von Tetrahydrobiopterin in Medium bzw. IL-2 versetzt. Die vorstehenden Präparate wurden 12 Stunden lang bei 37 °C und 5% $Co_2$ inkubiert. Zum Schluss wurde 3 Stunden mit je 74KBq $^3$H-Thymidin gepulst, geerntet und im β-Counter gezählt.

Die bei diesem Test erzielten Ergebnisse zeigten, dass bei alleiniger Zugabe zum Medium Tetrahydrobiopterin gegenüber den Indikatorzellen inaktiv ist. Eine Kombination von Interleukin-2 mit Tetrahydrobioptrin ergab eine Steigerung des $^3$H-Thymidin-Einbaues bis zum 5fachen vom Interleukin-2-Grundwert. Die Kombination verschiedener Konzentrationen von Interleukin-2 mit verschiedenen Konzentrationen von Tetrahydrobiopterin zeigte, dass eine optimale Kooperation ausgeprägt von der Konzentration beider Partner abhängt. Ein Wirkungoptimum wurde bei einer Tetrahydrobiopterin-Konzentration von $10^{-6}$ bis $10^{-5}$ Mol erzielt, bei einer Interleukin-2-Konzentration von 1 bis 20 Units/ml bezogen auf den vorläufigen Internationalen Standard.

Beispiel 2

A. Maus-Milz-Zellen wurden mit ConA gemäss dem in vorstehender Literaturstelle (9) beschriebenen Verfahren stimuliert.

B. Die gemäss Stufe A erhaltenen Zellen wurden wie in Beispiel 1, Stufe B, weiterbehandelt.

Die dabei erzielten Ergebnisse waren qualitativ identisch mit denjenigen des Beispiels 1.

Beispiel 3

Eine Interleukin-2-abhängige cytotoxische T-Zell-Indikatorlinie wurde in das gleiche Medium wie in Beispiel 1 B) beschrieben aufgenommen und wie in Beispiel 1 B) weiterbehandelt.

Die dabei erzielten Ergebnisse waren fast identisch mit denjenigen des Beispiels 1.

Beispiel 4

Man verfuhr wie in den Beispielen 1, 2 und 3 mit dem Unterschied, dass man jeweils hochreines Interleukin-2, rohes Interleukin-2 und inaktiviertes Interleukin-2 als Lymphokin-Präparat verwendete.

Die nachstehende Figur stellt eine Extrapolation sämtlicher Beispiele dar und bestätigt die im Zusammenhang mit Beispiel 1 gemachten Ausführungen.

Alle Arbeiten wurden wegen der Lichtempfindlichkeit des Tetrahydrobiopterins bei Rotlicht durchgeführt.

**Patentansprüche für die Vertragsstaaten BE, CH, DE, FR, GB, IT, LI, LU, NL, SE**

1. Verwendung einer Kombination aus Tetrahydrobiopterinen und Lymphokinen zur Herstellung eines Arzneimittels mit gesteigerter in vivo und in vitro Lymphokin-Aktivität.
2. Verwendung nach Anspruch 1, wobei das Lymphokin Interleukin-2 ist.
3. Verwendung nach einem der vorstehenden Ansprüche in einer Konzentration der Tetrahydrobiopterine von $10^{-6}$ bis $10^{-5}$ Mol.
4. Verwendung nach Anspruch 3 in einer Konzentration der Lymphokine von 1 - 20 Units/ml.
5. Diagnostisches und/oder therapeutisches Präparat auf der Basis von Lymphokinen, dadurch gekennzeichnet, dass es einen Zusatz eines Tetrahydrobiopterins enthält.
6. Präparat nach Anspruch 5, dadurch gekennzeichnet, dass das Lymphokin Interleukin-2 ist.
7. Präparat nach einem der Ansprüche 5 oder 6, dadurch gekennzeichnet, dass es $10^{-6}$ bis $10^{-5}$ Mol eines Tetrahydrobiopterins und 1 - 20 Units/ml eines Lymphokins enthält.

**Patentansprüche für den Vertragsstaat AT**

1. Verwendung einer Kombination aus Tetrahydrobiopterinen und Lymphokinen zur Herstellung eines Arzneimittels mit gesteigerter in vivo und in vitro Lymphokin-Aktivität.
2. Verwendung nach Anspruch 1, wobei das Lymphokin Interleukin-2 ist.
3. Verwendung nach Ansprüche in einer Konzentration der Tetrahydrobiopterine von $10^{-6}$ bis $10^{-5}$ Mol.
4. Verwendung nach Anspruch 3 in einer Konzentration der Lymphokine von 1 - 20 Units/ml.
5. Verfahren zur Herstellung eines diagnostischen und/oder therapeutischen Präparats auf der Basis von Lymphokinen, dadurch gekennzeichnet, dass man Tetrahydrobiopterine zusetzt.
6. Verfahren nach Anspruch 5, dadurch gekennzeichnet, dass man als Lymphokin Interleukin-2 verwendet.
7. Präparat nach einem der Ansprüche 5 oder 6, dadurch gekennzeichnet, dass es $10^{-6}$ bis $10^{-5}$ Mol eines Tetrahydrobiopterins und 1 - 20 Units/ml eines Lymphokins enthält.

**Claims for the contracting state AT**

1. The use of a combination of tetrahydrobiopterins and lymphokines for the preparation of a mediamcent showing enhanced in vivo and in vitro lymphokine activity.
2. The use claimed in claim 1, the lymphokine being 2-interleukin.
3. The use claimed in claim 3 in a concentration of the tetrahydrobiopterins of from $10^{-6}$ to $10^{-5}$ mol.
4. The use claimed in claim 3 in a concentration of the lymphokines of 1 to 20 units/ml.
5. A process for the production of a diagnostic and/or therapeutic preparation based on lymphokines, characterized in that tetrahydrobiopterins are added.
6. A process as claimed in claim 5, characterized in that the 2-interleukin is used asthe lymphokine.
7. A process as claimed in claim 5 or 6, characterized in that $10^{-6}$ to $10^{-5}$ mol of a tetrahydrobiopterin and 1 to 20 units/ml of a lymphokine are used.

**Claims for the contracting states BE, CH, DE, FR, GB, IT, LI, LU, NL, SE**

1. The use of a combination of tetrahydrobiopterins and lymphokines for the preparation of a mediamcent shwoing enhanced in vivo and in vitro lymphokine activity.
2. The use claimed in claim 1, the lymphokine being 2-interleukin.
3. The use claimed in any of the preceding claims in a concentration of the tetrahydrobiopterins of from $10^{-6}$ to $10^{-5}$ mol.
4. The use claimed in claim 3 in a concentration of the lymphokines of 1 to 20 units/ml.
5. A diagnostic and/or therapeutic preparation based on lymphokines, characterized in that it contains an addition of a tetrahdrobiopterin.
6. A preparation as claimed in claim 5, characterized in that the lymphokine is 2-interleukin.
7. A preparation as claimed in claim 5 or 6, characterized in that it contains $10^{-5}$ to $10^{-5}$ mol of a tetrahydrobiopterin and 1 to 20 units/ml of a lymphokine.

**Revendications BE, CH, DE, FR, GB, IT, LI, LU, NL, SE**

1. Application d'une combinaison de tétrahydrobioptérines et de lymphokines à la préparation d'un médicament à activité de lymphokine accrue in vivo et in vitro.
2. Application selon la revendication 1, où la lymphokine est l'interleukine-2.
3. Application selon l'une des revendications précédentes à une concentration des tétrahydrobioptérines de $10^{-6}$ à $10^{-5}$ moles.
4. Application selon la revendication 3, à une concentration de lymphokines de 1 à 26 unités/ml.
5. Préparation diagnostique et/ou thérapeutique à base de lymphokines, caractérisée en ce qu'elle contient une addition d'une tétrahydrobioptérine.
6. Préparation selon la revendication 5, caractérisée en ce que la lymphokine est l'interleukine-2.
7. Préparation selon l'une des revendications 5 ou 6, caractérisée en ce qu'elle contient $10^{-6}$ à $10^{-5}$ moles d'une tétrahydrobioptérine et de 1 à 20 unités/ml d'une lymphokine

**Revendications AT**

1. Application d'une combinaison de tétrahydrobioptérines et de lymphokines à la préparation d'un médicament à activité de lymphokine accrue in vivo et in vitro.

2. Application selon la revendication 1, où la lymphokine est l'interleukine-2.

3. Application selon l'une des revendications précédentes à une concentration des tétrahydrobioptérines de $10^{-6}$ à $10^{-5}$ moles.

4. Application selon la revendication 3, à une concentration de lymphokines de 1 à 20 unités/ml.

5. Procédé de préparation d'une préparation diagnostique ou thérapeutique à base de lymphokines, caractérisé en ce qu'on ajoute des tétrahydrobioptérines.

6. Procédé selon la revendication 5, caractérisé en ce qu'on utilise comme lymphokine l'interleukine-2.

7. Procédé selon l'une des revendications 5 ou 6, caractérisé en ce qu'on utilise $10^{-6}$ à $10^{-5}$ moles d'une tétrahydrobioptérine et de 1 à 20 unités/ml d'une lymphokine.

Fig.

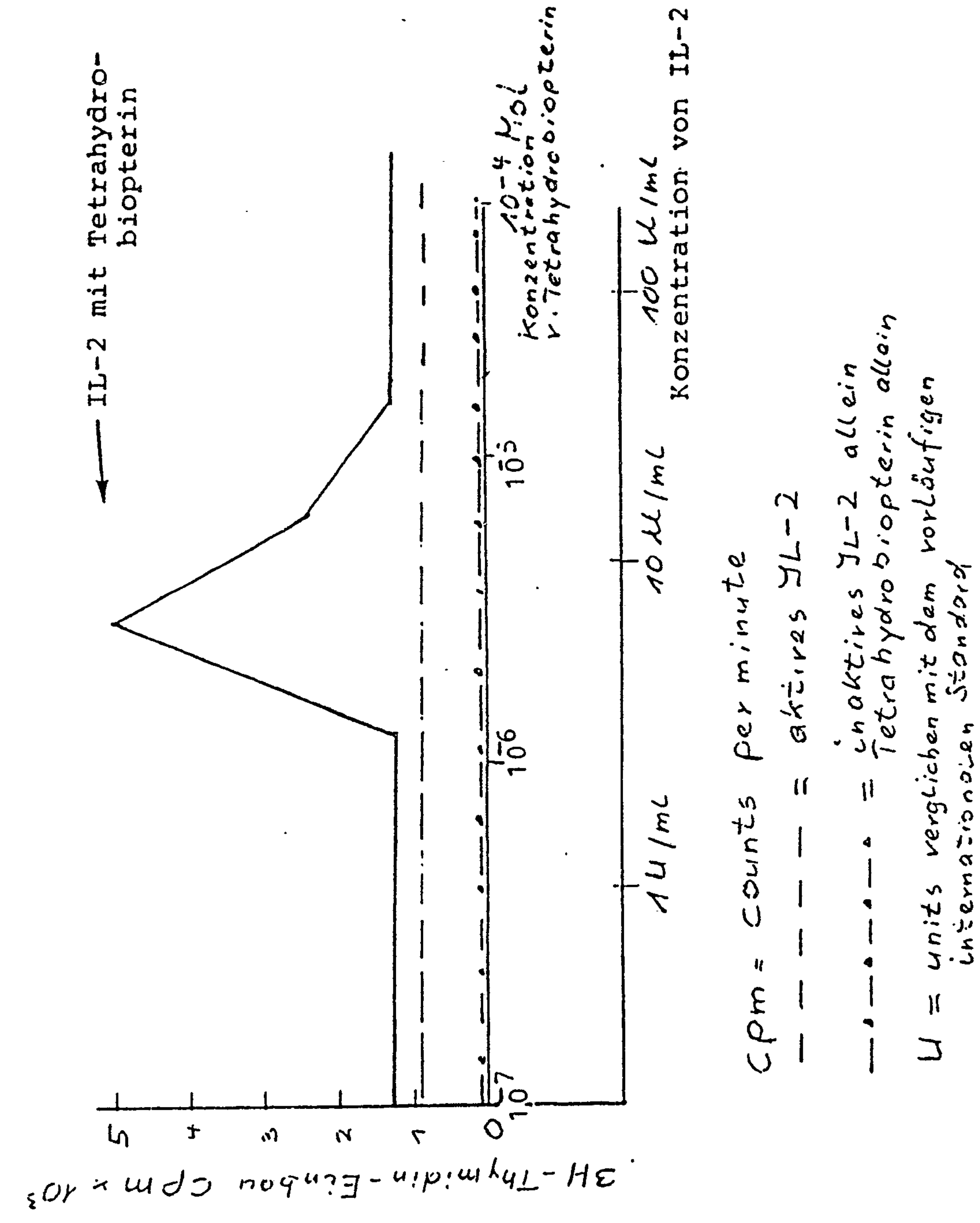
IL-2 mit Tetrahydro-
biopterin
3H-Thymidin-Einbau cpm x 10³
5
4
3
2
1
0
10⁻⁷
10⁻⁶
10⁻⁵
10⁻⁴ Mol
Konzentration
v. Tetrahydrobiopterin
1 U/ml
10 U/ml
100 U/ml
Konzentration von IL-2
cpm = counts per minute
— — — = aktives IL-2
—•—•—• = inaktives IL-2 allein
Tetrahydrobiopterin allein
U = units verglichen mit dem vorläufigen
internationalen Standard